(19) Europäisches Patentamt
European Patent Office
Office européen des brevets

(11) Publication number: **0 064 244**
**A1**

(12) **EUROPEAN PATENT APPLICATION**

(21) Application number: 82103468.3

(22) Date of filing: 23.04.82

(51) Int. Cl.³: C 07 D 233/58
A 61 K 31/415

(30) Priority: 24.04.81 US 256878

(43) Date of publication of application: 10.11.82 Bulletin 82/45

(84) Designated Contracting States: AT BE CH DE FR GB IT LI LU NL SE

(71) Applicant: SYNTEX (U.S.A.) INC.
3401 Hillview Avenue
Palo Alto, California 94304(US)

(72) Inventor: Walker, Keith A.M.
12798 Canario Way
Los Altos Hills California 94022(US)

(74) Representative: Schmied-Kowarzik, Volker, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

# (54) Spermicidal 1-bis(arylalkyl)alkylimidazoles.

(57) New compounds of the formula

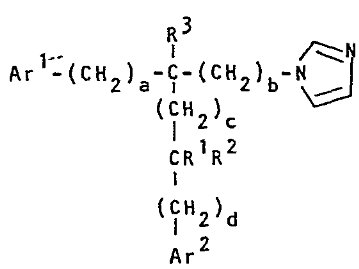

(1)

wherein:

$Ar^1$ and $Ar^2$ are each independently optionally substituted phenyl;

$R^1$, $R^2$ and $R^3$ are each independently hydrogen or alkyl;

a is an integer from 1-3;

b is an integer from 1-3;

c is an integer from 0-2; and

d is an integer from 0-2, such that (c + d) equals 0-2;

and the pharmaceutically acceptable non-toxic acid addition salts thereof, are spermicidal and, therefore, are useful as contraceptives.

EP 0 064 244 A1

## BACKGROUND OF THE INVENTION

This invention concerns certain 1-substituted imidazoles which are useful as antifungals, uterine relaxants, and, most particularly, as spermicides effective when administered either to male or female mammals.

Vast numbers of 1-substituted imidazole compounds are known. Various members of this group have been described as, for example, antifungals, antibacterials, anticonvulsants, food preservatives, crop disease protecting agents and CNS agents. U.S. Patent 4,247,552, issued January 27, 1981 and incorporated herein by reference discloses 1-substituted imidazoles which are spermicides.

Compounds closest in structure to those of the present invention are disclosed in U.S. 3,991,201, 3,927,017 and 4,115,578; and in Belgian 848,004 and 827,870. In general, these patents describe compounds which are straight chain alkanes with two aryl substituents in addition to the 1-imidazolyl substituent which relates them to the compounds of the present invention.

It has now been found that certain simple or complex branched chain alkanes which are herein described,

substituted on the terminal carbon atoms with two optionally substituted phenyl groups and with one 1-imidazolyl group, each separated from the branch carbon by at least one methylene unit, have surprisingly potent spermicidal activity.

## SUMMARY OF THE INVENTION

The present invention relates to compounds of the formula

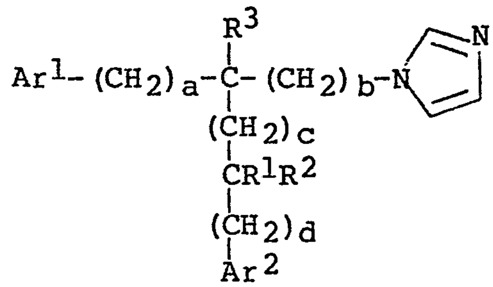

(I)

wherein:

$Ar^1$ and $Ar^2$ are each independently optionally substituted phenyl;

$R^1$, $R^2$ and $R^3$ are each independently hydrogen or alkyl;

a is an integer from 1-3;

b is an integer from 1-3;

c is an integer from 0-2; and

d is an integer from 0-2, such that (c + d) equals 0-2;

and the pharmaceutically acceptable non-toxic salts thereof.

In two other aspects, the invention concerns contraceptive pharmaceutical compositions containing compounds of formula I as active ingredients, and a method of contraception utilizing the spermicidal properties of said compounds.

The invention also relates to a process for preparation of compounds of formula I.

## DETAILED DESCRIPTION

Definitions:

As used herein, "alkyl" means a branched or unbranched saturated hydrocarbon residue containing 1, 2, 3 or 4 carbons, such as for example, methyl, ethyl, n-propyl, i-propyl, n-butyl, i-butyl, or t-butyl.

"Alkoxy" means -OR, wherein R is alkyl as herein defined;

"Halo" means fluoro, chloro or bromo;

"Pharmaceutically acceptable acid addition salt" refers to those salts which qualitatively retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

"Optional" or "optionally" means that the subsequently described event or circumstance may or may not occur, and that the description includes instances where said event or circumstance occurs and instances in which it does not. For example, "optionally substituted phenyl" means that the phenyl may or may not be substituted (see below for substitution definitions) and that the description includes both unsubstituted phenyl and phenyl wherein there is substitution; "optionally followed by converting the free base to the acid addition salt" means that said conversion may or may not be carried out in order for the process described to fall within the invention, and the invention includes those

processes wherein the free base is converted to the acid addition salt and those processes in which it is not.

"Substituted phenyl" as used herein means that one or more hydrogens of the phenyl ring are replaced by moieties selected from the group consisting of halo (see above definition), alkyl (see above definition), alkoxy or trifluoromethyl. In the context of the present invention, said replacement may be at any position of the phenyl ring, and 1, 2, 3, 4, and/or 5 hydrogens may be so replaced. However if more than three hydrogens are replaced, all substituents must be identical.

"Spermicide" and "spermicidal" refer to the capacity to render spermatozoa ineffective. This effect may be the result of actual sperm cell death, or less drastically, immobility, cell membrane alteration or other impairment which results in the inability of the sperm cell to effect fertilization.

Process And Preparation:

General Parameters

All of the compounds of the invention contain at least one chiral center, i.e., the carbon to which $R^3$ is attached (except for the special case in which the two phenyl containing side chains are identical, in which case the following remarks, of course, do not apply).

Accordingly, the compounds of the present invention may be prepared in either optically active form, or as a racemic mixture. Unless otherwise specified, the compounds described herein are all in the racemic form. However, the scope of the subject invention herein is not to be considered limited to the racemic form, but to encompass the individual optical isomers of the subject compounds.

If desired, racemic intermediates or final products prepared herein may be resolved into their optical antipodes by conventional resolution means known per se,

for example, by the separation (e.g., fractional crystallization) of the diastereomeric compounds formed by reaction of racemic mixtures with optically resolved reactants.

Racemic compounds of Formula I and other basic intermediates may be resolved by separation of the diastereomers formed by reaction with optically active acids. Exemplary of such optically active acids are the optically active forms of camphor-10-sulfonic acid, α-bromocamphor-π-sulfonic acid, camphoric acid, menthoxyacetic acid, tartaric acid, malic acid, diacetyltartaric acid, pyrrolidone-5-carboxylic acid and the like.

Resolution of the enantiomers of the compounds of formula II (infra) may be accomplished by separation of the diastereomeric esters or carbamates formed by reaction with optically active acids or isocyanates. Exemplary of such optically active isocyanates are 1-(α-naphthyl)ethyl isocyanate, menthyl isocyanate, and 1-phenylethyl isocyanate.

Carboxylic acid intermediates such as those of formula IV, may also be resolved, for example, through formation of and separation of their diastereomeric salts, formed by reaction with optically active amines, or esters formed with optically active alcohols. Typical of such amines and alcohols are, for example, the optically active forms of brucine, dehydroabietylamine, ephedine, α-phenethylamine, 1-(1-naphthyl)ethylamine, and menthol. Under different conditions, the above mentioned amines may be used to form the corresponding amides.

The separated pure diastereomeric compounds described above may then be cleaved by standard means to afford the respective optical isomers of the racemic compound subjected to resolution.

Compounds of the present invention, or intermediates

in the preparations thereof, described hereinbelow, may be isolated and purified by conventional means known in the art, such as fractional crystallization, extraction and solvent removal, high pressure liquid chromatography, column choromatography, thin layer chromatography and the like. The degree of purification required for any intermediate in the preparation sequences will, of course, depend on the nature of contaminants, and the selectivity of the subsequent reagents. The salt products are also isolated by conventional means. For example, the acidified reaction mixture containing the compound of formula I, may be evaporated to dryness, and the salts can be further purified by usual methods. Isolation of such salts is often more convenient, as many of the compounds of the invention, as free bases, are oils.

The compounds of formula I and the intermediates described hereinbelow are represented in expanded form everywhere herein for ease of reading. Such representations are <u>not</u> intended to be Fischer projections, and no particular configuration should be inferred from these representations.

<u>Process</u>

Compounds of formula I are prepared according to Reaction Scheme A:

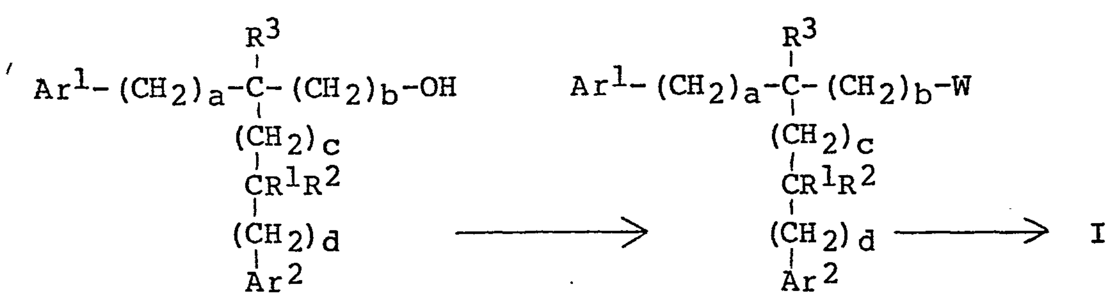

II III

<u>REACTION SCHEME A</u>

W represents a leaving group, such as, for example, a halide (chloro, bromo or iodo) or reactive ester, (for example a p-tolulenesulfonate or methanesulfonate ester).

The conversion of II to III is accomplished by treating a compound of formula II with either a halogenating agent, such as, for example, thionyl bromide or N-bromosuccinimide/triphenylphosphine or with an appropriate sulfonyl halide, for example p-toluenesulfonyl chloride, optionally in an inert solvent, such as, for example dichloromethane or tetrahydrofuran (THF), preferably THF, in the presence of base, e.g. a tertiary amine, such as, pyridine or triethylamine. The base itself, for example, pyridine, may also be used as solvent. The reaction is carried out at a temperature of about -20° to about 50°C preferably 0°-25°C and over a period of 5 minutes to 24 hours, preferably 30 minutes to overnight. Thereafter, the compound of formula III is converted to the final product of formula I, by treating it with at least one mole of imidazole per mole of III, preferably an excess of 1.5 to 5 moles of imidazole per mole of III. The reaction takes place either in the absence of solvent (i.e. above the m.p. of the mixture) or in an inert organic solvent such as dimethylformamide (DMF) acetonitrile, tetrahydrofuran, or dimethylsulfoxide (DMSO); preferably, DMF, at a temperature between about 0° to 170°C, most preferably from about 50° to 150°C. Alternatively, the reaction may be carried out using a salt of imidazole, for example, an alkali metal salt, preferably a sodium salt, in the same solvents, at a temperature from about 0° to 150°C, preferably from about 20° to about 110°C.

Preparation of Compounds of Formula II

The compounds of formula II are prepared by one of several reaction schemes, as described hereinbelow. In

the representations of said reaction schemes, $R^4$ represents $Ar^1(CH_2)_a-$, $R^5$ represents $Ar^2(CH_2)_dCR^1R^2(CH_2)_c-$, R is alkyl, and $R^3$ and W are as herein defined.

A. <u>Compounds of Formula II Wherein b = 1</u>

The compounds of formula II wherein b is equal to 1 ($II_1$) are conveniently prepared by reduction of the corresponding carboxylic acids, which in turn, can be prepared by a number of methods known to those skilled in the art, such as, for example, malonic ester synthesis, alkylation of carboxylic acids or esters, and by the alkylation of β-keto esters, Meldrums acid, nitriles, or amides, and the like, preferably by malonic ester synthesis, optionally followed by alkylation of the resulting acid or ester. Exemplary of such a process is that shown in Reaction Scheme B:

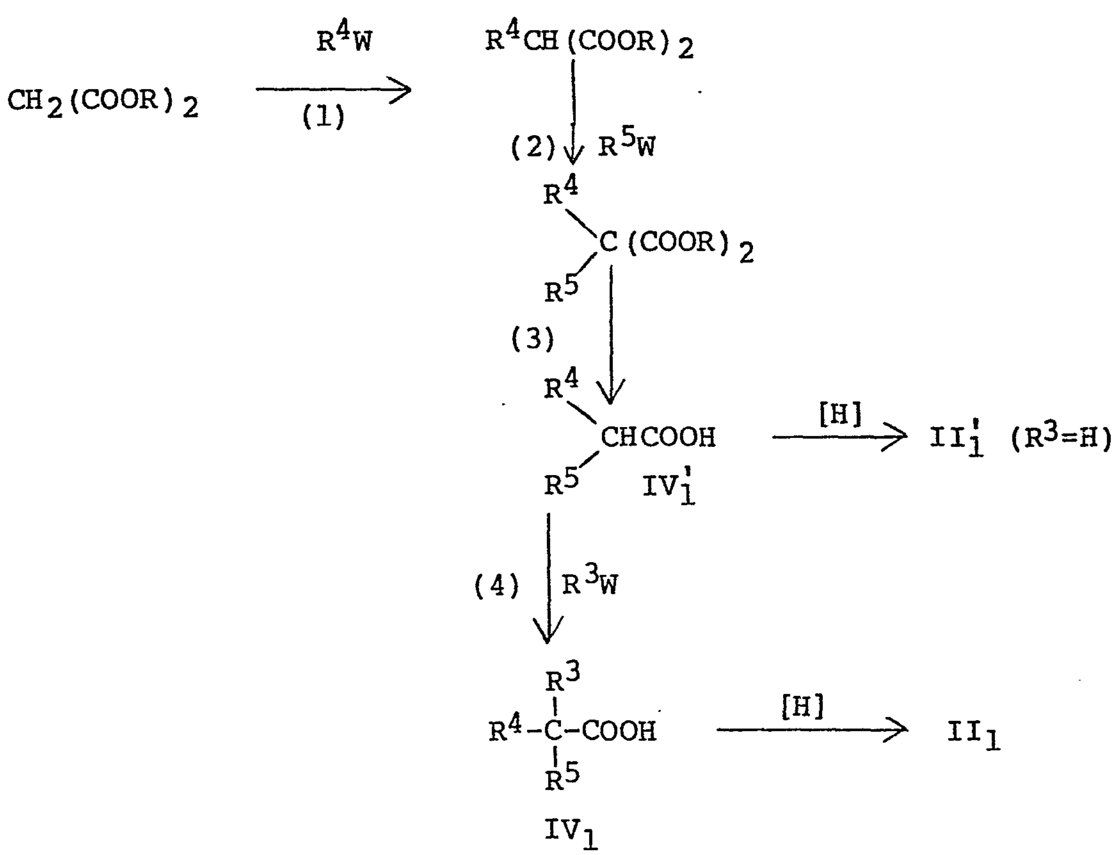


<u>REACTION SCHEME B</u>

The sequence of the alkylation steps may, of course, be done in any order, and either $R^3$, $R^4$ or $R^5$ is added first. The required reaction conditions for steps (1) to (3) are well-known to those skilled in the art.

The conversion of the acid of formula $IV_1'$ to the acid of formula $IV_1$ may be carried out by alkylating $IV_1'$ either as a dianion (e.g. the dilithium derivative) or as as an ester anion (e.g. the t-butyl ester derivative). This is then followed by hydrolysis to give a compound $IV_1'$, by known methods.

The compound of formula $IV_1$ or $IV_1'$ is then reduced to the corresponding compound of formula $II_1$ using an appropriate reducing agent, such as, for example, borane/tetrahydrofuran complex or lithium aluminum hydride.

In addition certain compounds of formula II may be obtained by reduction, e.g. with sodium borohydrate, of an aldehyde of the formula

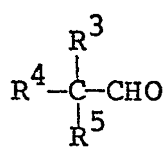 (V)

wherein $R^3$, $R^4$ and $R^5$ are as herein defined. Aldehydes of formula V may be prepared by methods known in the art, e.g. by alkylation of a simpler aldehyde or aldehyde derivative, especially the corresponding enamine.

B. <u>Compounds of Formula II Wherein b is Greater than 1</u>

Compounds of formula II wherein b is greater than 1, may be prepared by homologation of compounds of formula $II_1$ or from their more highly oxidized cogeners. Exemplary of such homologations are the sequences of reactions shown in Reaction Schemes C and D.

Reaction Scheme C, as shown below, results in a

compound of formula II wherein b=3($II_3$):

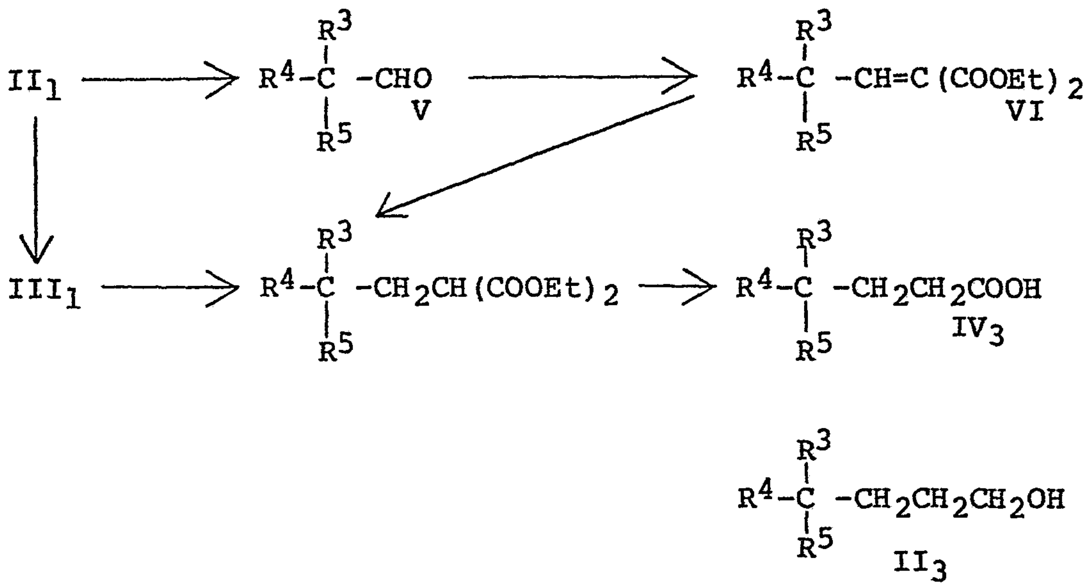

REACTION SCHEME C

In Reaction Scheme C, the compound of formula $II_1$ is converted to its halide or sulfonate of formula $III_1$using conditions previously described. The compound of formula $III_1$ is then converted to the corresponding malonic ester and then to the carboxylic acid of formula $IV_3$ in a manner analogous to that described in Reaction Scheme B for the formation of $IV_1'$.

Alternatively the acid of formula $IV_3$ is prepared from the aldehyde of formula V, which can be prepared by oxidation of $II_1$ by well known methods, for example, oxidation with DMSO/oxalyl chloride. The aldehyde of formula V is then condensed to give an olefin of formula VI in a Knoevenagel reaction. Reduction by convential means gives the corresponding saturated diester, which is then converted to the acid of formula $IV_3$.

Reaction Scheme D, results in a compound of formula II wherein b=2($II_2$), or may be repeated to generate a compound of formula II wherein b=3($II_3$):

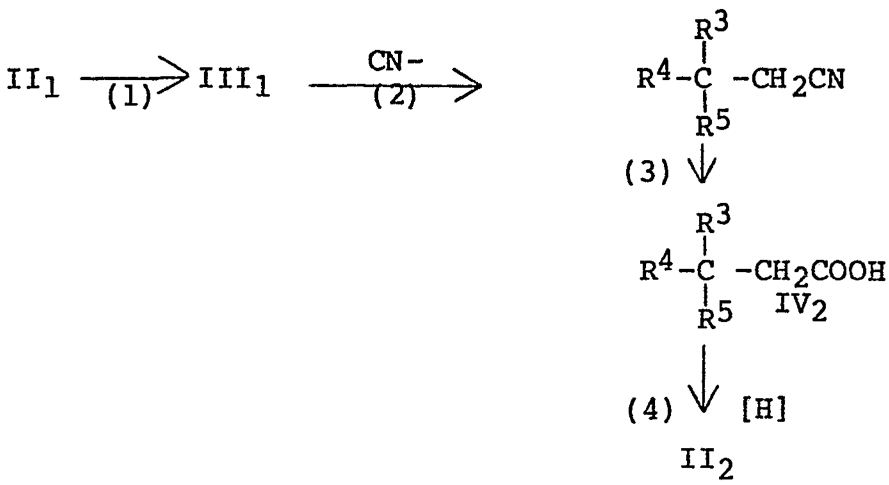


REACTION SCHEME D

The conditions and reagents for steps (1) and (4) of Reaction Scheme D have been described previously herein. Steps (2) and (3) are conventional in the art. Step (2) is conveniently done using sodium cyanide in DMSO or DMF; the hydrolysis of step (3) is conventionally carried out in the presence of a strong inorganic aqueous acid, such as, for example, sulfuric acid.

Reaction Scheme D is exemplary of one-carbon homologation methods known to those skilled in the art. Other such methods include, for example the Arndt-Eistert Synthesis and Wolff Rearrangement which convert the corresponding acids of formula $IV_1$ to the homologous acids of formula $IV_2$. Again, the resulting acid is reduced to $II_2$ which can, of course, then be resubjected to Reaction Scheme D to yield the corresponding compound of formula $II_3$.

C. Particular Methods for Compounds of Formula $II_2$

Reaction Schemes E and F represent useful methods for preparing certain compounds of formula $II_2$:

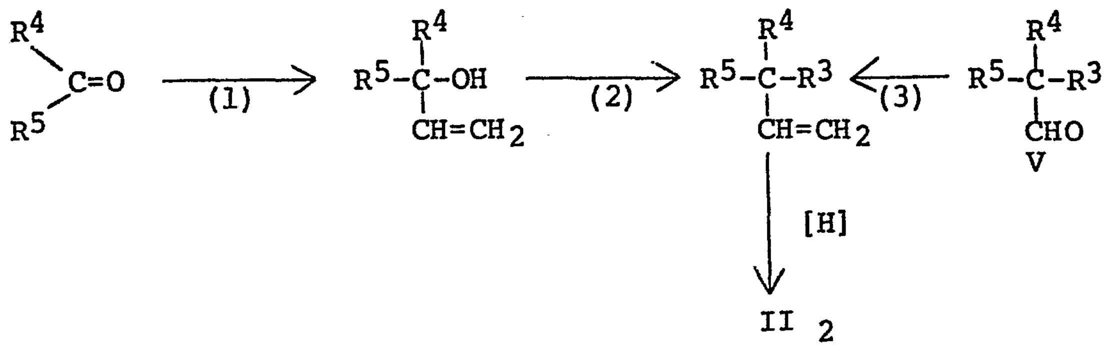

REACTION SCHEME E

The starting material, $R^4R^5C=O$ is prepared by known methods, for example by alkylation of a dithiane derivative followed by hydrolysis. However, most such starting materials are either commercially available, or known in the art, or both. The conversion to the vinyl carbinol in step (1) is effected by known methods, for example, by reaction with vinyl lithium or vinyl magnesium chloride. Subsequent replacement of hydroxy with $R^3$ is accomplished by reaction with methyl lithium in the presence of a palladium complex according to the method described in J. Amer. Chem. Soc.: 100:6445 (1978). (Alternatively the olefin prepared in step (2) may be prepared by Wittig olefination of the aldehyde, V, in a conventional procedure.) Standard techniques of hydroboration and oxidation yield the product compound of formula $II_2$.

Reaction Scheme F shows another method for the preparation of certain alcohols of formula $II_2$.

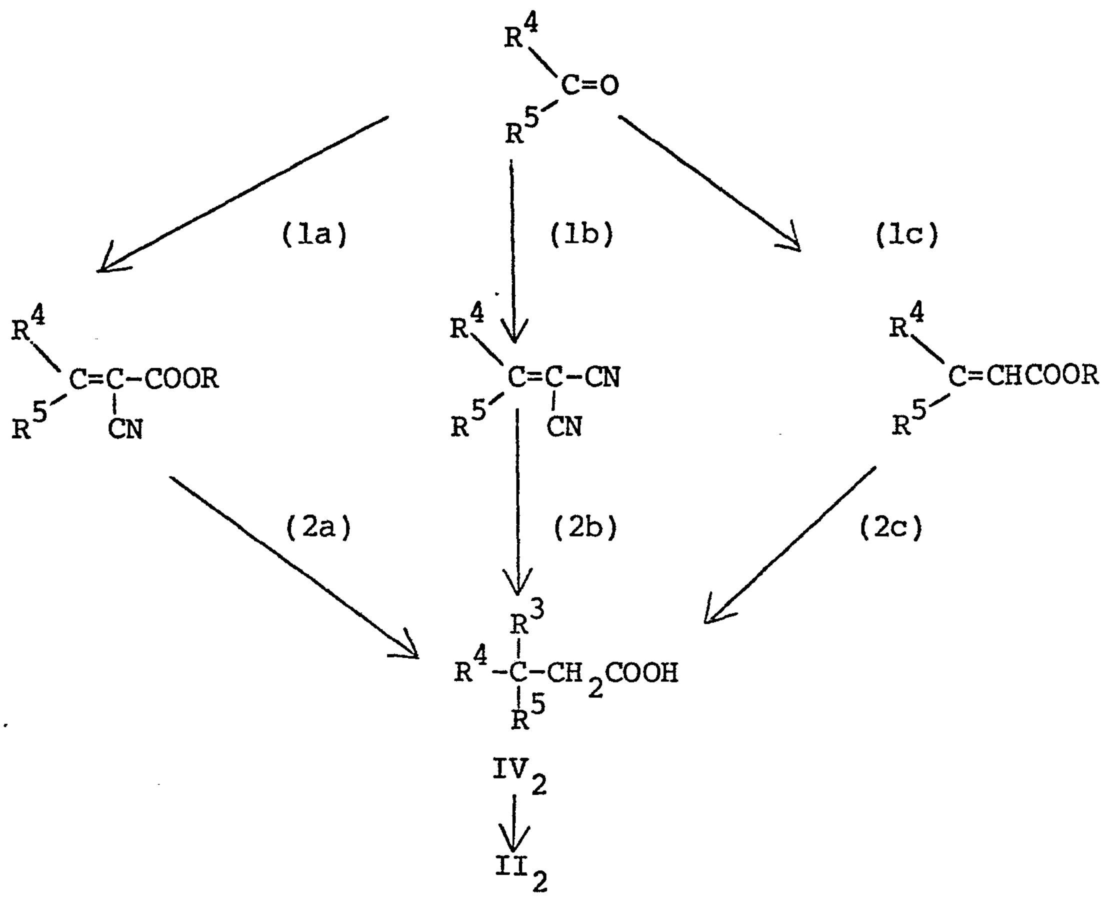

REACTION SCHEME F

Steps 1a, 1b and 1c are well known in the art. For example, using condensation with cyanoacetic ester or malononitrile in 1a or 1b respectively constitute practical methods; step 1c is by Wittig, (Horner-Emmons) olefination using a phosphorane (or phosphonate anion) or using a trialkylsilylacetic ester derivative.

To form the corresponding carboxylic acids of formula $IV_2$ wherein $R^3$ is hydrogen, hydrogenation of the double bond is effected in steps 2a, 2b or 2c by conventional means,followed by hydrolysis and pyrolysis as appropriate. Alternatively, if $R^3$ is alkyl, appropriate reagents such as, for example, dialkyl copper lithium reagent or alkyl Grignard reagent followed by hydrolysis and pyrolysis may be used. See, for example, Tetrahedron

Letters, 1967: 2393; ibid 1964: 1763.

Subsequent hydrogenation of the compound of formula $IV_2$ to the corresponding alcohol of formula $II_2$ has previously been described.

Alternative Overall Synthesis

Reaction Scheme G offers a method for embellishment of a preformed imidazole ketone with an additional substituent at the incipient branch point, for those compounds wherein $R^3$ is equal to H.

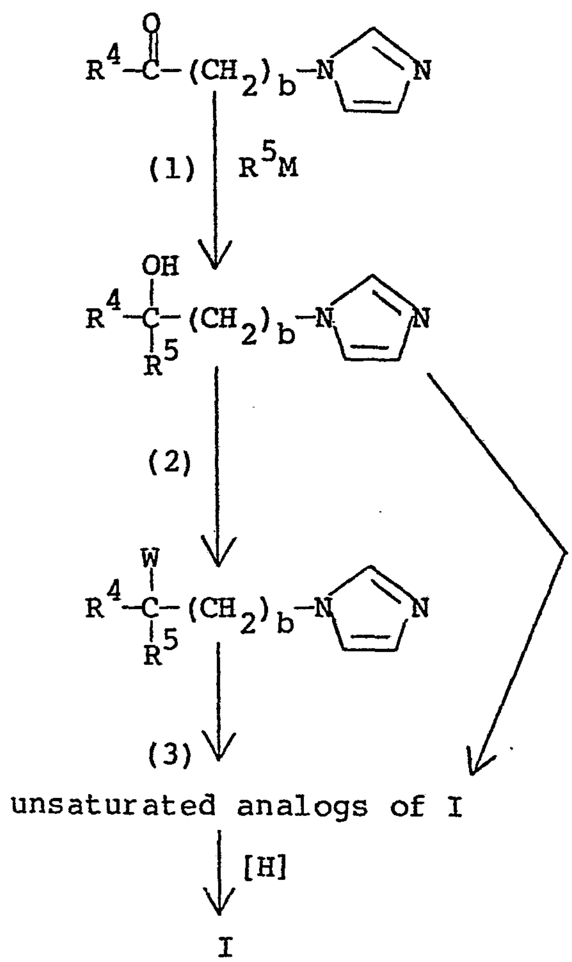


REACTION SCHEME G

(M, herein, represents a substituent metal ion or metal salt ion, for example, -Li or -MgBr.)

"Unsaturated analogs of I" represents one or more of the compounds of the formula:

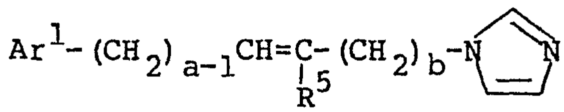 (A)

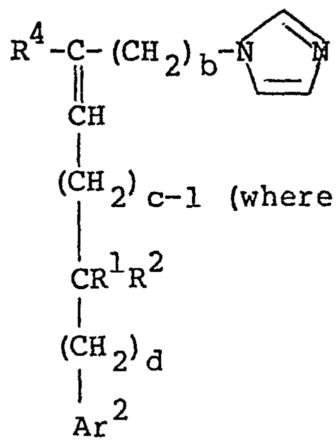 (B)

(where c≠0)

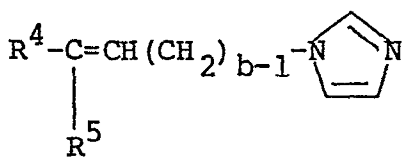 (C)

wherein $Ar^1$, $Ar^2$, $R^1$, $R^2$, $R^4$, $R^5$, a, b, c, and d are as herein defined except that in the compound of formula B, c=0.

The starting imidazole ketones are prepared according to the methods described in U.S. 4,078,071.

The conversion to the alcohol in step (1) is effected with an arylalkyllithium or arylalkyl magnesium bromide reagent wherein the arylalkyl group is the $R^5$ or $R^4$ moiety desired to be added. (It is understood that the reagents in step (1) are interchangable, i.e., the imidazole ketone may be substituted with $R^5$ and the alkyl metal compound contain the alkyl group represented by $R^4$ rather than the exact sequence shown above.)

Conditions and reagents for step (2) of Reaction Scheme G have been hereinbefore described.

The halide or sulfonate formed in step (2) can be directly reduced using appropriate reagents to the compound of formula I wherein $R^3$ is equal to hydrogen. Alternatively a preliminary elimination of either the hydroxyl group of the alcohol or the leaving group of the halide or sulfonate and hydrogen from an adjacent carbon (either from $(CH_2)_b$, $R^4$, or $R^5$, results in one or more isomeric elimination products which can subsequently be reduced by hydrogen with appropriate catalysts, to the compounds of formula I, again wherein $R^3$ is equal to hydrogen. (This method is most useful for those compounds wherein the aryl groups are not substituted by halo.)

An alternative sequence utilizing the same imidazole ketone starting materials is Wittig olefination, followed by reduction. Again, only those compounds of formula I wherein $R^3$ is hydrogen are obtained by this method.

In both of the above sequences (Reaction Scheme G and the Wittig olefination of the previous paragraph) the final step is the hydrogenation of a double bond. To carry out this step, the unsaturated analog of the compound of formula I (or mixture as indicated above) is dissolved in an inert solvent such as methanol, ethanol, acetone, acetic acid and the like, preferably ethanol containing acetic acid, and about 1% to 20% by weight, preferably about 10% by weight of hydrogenation catalyst, such as 10% palladium or charcoal or platinum oxide, preferably 10% palladium or charcoal, is added. The mixture is treated with an excess of hydrogen at ambient pressure, at about 10°C-30°C, preferably room temperature, until the uptake of hydrogen ceases. The resulting compound of formula I is then isolated by

conventional means.

Preferred Embodiments

Each of the preferred embodiments disclosed hereinbelow includes both the free base form and the pharmaceutically acceptable acid addition salts.

A set of preferred embodiments of the compounds of the invention is that wherein $R^1$, $R^2$ and $R^3$ are each independently methyl or hydrogen and each phenyl ring of $Ar^1$ and $Ar^2$ is independently unsubstituted or substituted with substituents selected from the group consisting of halo, alkyl and methoxy. When more than three substituents are present in the ring, it is preferred that these are chloro or methyl.

A more preferred set of embodiments is that wherein $R^1$, $R^2$ and $R^3$ are all hydrogen, and each phenyl ring is independently either not substituted, or substituted with one to two substituents selected from the group consisting of fluoro, chloro, methyl or methoxy; b is 1 or 2, c is 0 or 1, and d is 0 or 1. Especially preferred within this class are those compounds wherein a = 2 or 3.

Still more preferred are those embodiments wherein $Ar^1$ is selected from the group consisting of phenyl, 4-chlorophenyl, 4-fluorophenyl, 4-methylphenyl, 4-methoxyphenyl; $Ar^2$ is selected from the group consisting of phenyl, 2-chlorophenyl, 4-chlorophenyl, 2,4-dichlorophenyl, 2-methylphenyl, 4-methylphenyl, 2,4-dimethylphenyl, 2-methoxyphenyl, or 4-methoxyphenyl; a is 2, b is 1, and (c + d) is 1 or 0.

A most preferred set of embodiments is that wherein the compound of formula I is selected from the group consisting of

1-[4-(4-chlorophenyl)-2-(2-phenylethyl)-n-butyl]-imidazole;

1-[4-(4-chlorophenyl)-2-(4-fluorobenzyl)-n-butyl]-imidazole;

1-[4-(4-chlorophenyl)-2-(2-chlorobenzyl)-n-butyl]-imidazole;

1-[4-(4-chlorophenyl)-2-(2-(2-chlorophenyl)ethyl)-n-butyl]imidazole;

1-[4-(4-chlorophenyl)-2-(2-(4-chlorophenyl)ethyl)-n-butyl]imidazole;

1-[4-(4-chlorophenyl)-2-(4-chlorobenzyl)-n-butyl]-imidazole;

1-[4-(4-chlorophenyl)-2-(2,4-dichlorobenzyl)-n-butyl]imidazole;

1-[4-(4-chlorophenyl)-2-(3.4-dichlorobenzyl)-n-butyl]imidazole.

Utility and Administration

The compounds of formula I herein and the pharmaceutically acceptable non-toxic acid addition salts thereof are primarily useful as spermicides, either intravaginally administered to the female mammal or orally or parenterally administered to the male mammal.

Compositions appropriate for such uses are prepared by methods and contain ingredients which are well known in the art. A generally recognized compendium of such methods and ingredients is Remington's Pharmaceutical Sciences by E. W. Martin, (Mark Publ. Co., 15th Ed., 1975).

For intravaginal administration suitable formulations are, for example creams, gels, spray foams, suppositories and the like, as well as slow release materials. Each composition contains an effective amount of active ingredient plus one or more pharmaceutically acceptable excipients. Such excipients are, for example, starch, glucose, lactose, talc, cellulose and the like for solid formulations; polyethylene glycols, modified vegetable oils, mineral oil, or polyalkylene glycols and the like for semi-solid formulations; and water, alcohols, glycerol, lanolin, mineral oil and the like for

liquid or semi-liquid compositions. The compositions may contain between about 0.01 and 10.0 percent by weight of the active ingredient, preferably between 0.1 and 1.0%, and may, if desired, contain other active ingredients. Such compositions may also be used in conjunction with barrier methods such as, e.g., diaphragms or condoms.

In the practice of the method of contraception herein, the above formulations are administered to the female before coitus, within a period of about 12 hours prior thereto. The preferred dosage range of active ingredient is from about 1 mg to 20 mg per vaginal administration for an adult human. For smaller mammals the amount would be correspondingly smaller.

Compositions for administration to the male are preferably directed to oral administration, although parenteral administration is also biologically possible. Such compositions will contain a spermicidal amount of active ingredient with a non-toxic, pharmaceutically effective carrier. For said oral administration solid dosage forms such as tablets, capsules and powders may contain such excipients as, for example, lactose, starch, or cellulose.

In the practice of the method of contraception herein, a dose in the range of between about 0.1 and 10 mg active ingredient per kg will be administered to the male prior to coitus, preferably at least 24 hours before coitus, and more preferably daily for 3-7 days prior to coitus.

The compounds of the present invention also exhibit other properties: antifungal/antibacterial activities, and antithromboxane and uterine relaxant capabilities.

The following examples are illustrative of the methods and compositions of the present invention. They should not be construed as limitative thereof in any manner.

## PREPARATION 1

### Diethyl 2-[2-(4-chlorophenyl)ethyl]malonate

A. Diethyl malonate (32 g) was added to a suspension of sodium hydride (10.56 g of a 50% dispersion in mineral oil) in 200 ml of dry tetrahydrofuran under nitrogen at a rate such as to maintain gentle reflux. The mixture was then heated under reflux for another hour and then treated with 2-(4-chlorophenyl)-1-bromoethane (48.3 g) and potassium iodide (10 g), under reflux for two days with stirring. The reaction mixture was poured into 400 ml ice water and extracted with 2 x 400 ml of ethyl acetate. The combined extracts were dried ($MgSO_4$), evaporated and the residue chromatographed on silica gel eluting with 30% dichloromethane/hexane to give 38 g of pure diethyl 2-[2-(4-chlorophenyl)ethyl]malonate as an oil.

B. Similarly, substituting into the procedure of Part A hereinabove, for 2-(4-chlorophenyl)-1-bromoethane, the following:

2-phenyl-1-bromoethane;
3-(2-chlorophenyl)-1-bromopropane;
3-(2,4-dichlorophenyl)-1-bromopropane;
4-fluorobenzyl bromide;
2-(4-methylphenyl)-1-bromoethane;
2-(4-fluorophenyl)-1-bromoethane; or
3-(3-chlorophenyl)-1-bromopropane;

one obtains

1. diethyl 2-(2-phenylethyl)malonate;
2. diethyl 3-(3-(2-chlorophenyl)propyl)malonate;
3. diethyl 2-(2-(2,4-dichlorophenyl)propyl)malonate;
4. diethyl 2-(4-fluorobenzyl)malonate;
5. diethyl 2-(2-(4-methylphenyl)ethyl)malonate;
6. diethyl 2-(2-(4-fluorophenyl)ethyl)malonate;
7. diethyl 2-(3-(3-chlorophenyl)propyl)malonate.

PREPARATION 2

Diethyl 2-[2-(4-chlorophenyl)ethyl]-2-(2-phenylethyl)malonate

A. Diethyl 2-[2-(4-chlorophenyl)ethyl]malonate (3.24 g) was added dropwise to a stirred suspension of sodium hydride (0.62 g of a 50% dispersion in mineral oil) in 20 ml of dry tetrahydrofuran under nitrogen. After stirring for another hour at about 50°C, 2-phenethylbromide (2.4 g) was added and the mixture heated overnight under reflux under nitrogen. The reaction mixture was then poured into 250 ml of ice water, extracted with a total of 350 ml of ethyl acetate and the extracts dried ($MgSO_4$) and evaporated to give crude diethyl 2-[2-(4-chlorophenyl)ethyl]-2-(2-phenylethyl)malonate as a yellow oil (5.4 g).

B. Similarly, substituting into the procedure of Part A hereinabove, for diethyl 2-[2-(4-chlorophenyl)ethyl]-malonate, each of the seven malonate derivatives set forth in part B of Preparation 1, with each correspondingly numbered bromide in the following:

1. 3-(4-methylphenyl)propyl bromide;
2. 2-(4-methoxyphenyl)ethyl bromide;
3. 3-(3-chlorophenyl)propyl bromide;
4. 4-fluorobenzyl bromide;
5. 1-(2,4-dimethylphenyl)-1-bromoethane;
6. 2-methyl-2-(4-methoxyphenyl)-1-bromobutane;
7. 2-methyl-1-(2-chlorobenzyl)-2-bromobutane;

one obtains, respectively,

1. diethyl 2-(2-phenylethyl)-2-(3-(4-methylphenyl)-propyl)malonate;
2. diethyl 2-(3-(2-chlorophenyl)propyl-2-(2-(4-methoxyphenyl)ethyl)malonate;
3. diethyl 2-(3-(2,4-dichlorophenyl)propyl)-2-(3-(3-chlorophenyl)propyl)malonate;

4. diethyl 2-(4-fluorobenzyl)-2-(4-fluorobenzyl)-malonate;
5. diethyl 2-(2-(4-methylphenyl)ethyl)-2-(1-(2,4-dimethylphenyl)ethyl)malonate;
6. diethyl 2-(2-(4-fluorophenyl)ethyl)-2-(2-(4-methoxy-phenyl)-2-methylbutyl)malonate;
7. diethyl 2-(3-(3-chlorophenyl)propyl)-2-(1-(2-chloro-benzyl)-1-methylpropyl)malonate.

## PREPARATION 3

### 4-(4-chlorophenyl)-2-(2-phenylethyl)butan-1-ol

A. (1) The diester prepared in Part A of Preparation 2 (5.4 g) was heated under reflux overnight with stirring with a mixture of potassium hydroxide (25 g) and methanol (25 ml) in water (25 ml). After evaporation of most of the methanol under reduced pressure, the mixture was poured into 200 ml of water and extracted with ethyl acetate. The aqueous phase was then acidified with ice cold dilute hydrochloric acid, extracted twice with ethyl acetate, and the extracts dried ($MgSO_4$) and evaporated. The resulting pink solid (2.5 g), was decarboxylated by heating under nitrogen at 175°C overnight, to give 4-(4-chlorophenyl)-2-(2-phenylethyl)butyric acid used directly in the next step.

(2) The acid from above was dissolved in 20 ml of dry tetrahydrofuran and treated dropwise with stirring at room temperature with 10.65 ml of 1.06 M borane:tetrahydrofuran solution. After two hours, the yellow solution was poured into ice water, basified with aqueous potassium carbonate and the mixture extracted with ethyl acetate (2 x 100 ml). The combined extracts were dried ($MgSO_4$) and evaporated to give 2.39 g of the title compound.

B. Similarly, substituting into the procedure of Part A hereinabove, for diethyl 2-[2-(4-chlorophenyl)ethyl]-2-(2-phenylethyl)malonate the malonate diesters listed in part B of preparation 2, one obtains, respectively:

1. 4-phenyl-2-(3-(4-methylphenyl)propyl)butan-1-ol;
2. 5-(2-chlorophenyl)-2-(2-(4-methoxyphenyl)ethyl)-pentan-1-ol;
3. 5-(2,4-dichlorophenyl)-2-(3-(3-chlorophenyl)-propyl)-pentan-1-ol;
4. 3-(4-fluorophenyl)-2-(4-fluorobenzyl)propan-1-ol;
5. 4-(4-methylphenyl)-2-(1-(2,4-dimethylphenyl)-ethyl)-butan-1-ol;
6. 4-(4-fluorophenyl)-2-(2-(4-methoxyphenyl)-2-methyl-butyl)butan-1-ol;
7. 5-(3-chlorophenyl)-2-(1-(2-chlorobenzyl)-1-methyl-propyl)pentan-1-ol.

## PREPARATION 4

## 4-(4-chlorophenyl)-2-(2-phenylethyl)-2-(ethyl)-n-butan-1-ol

A. The procedure is based on the method disclosed in J. Org. Chem., 37, 451 (1972).

A mixture of 20 ml of dry tetrahydrofuran and diisopropylamine (2.25g) is purged with nitrogen and cooled to -20°C, whereupon a 1.5 M solution of n-butyllithium in hexane (14.6 ml) is added with stirring such that the temperature does not exceed 0°C. A solution of 4-(4-chlorophenyl)-2-(2-phenylethyl) butyric acid [as prepared in Part A (1) of Preparation 3] (3.02g) in a few ml of dry tetrahydrofuran is then added, maintaining the temperature below 0°C, and the resulting mixture stirred at 50°C for 2 hours, and cooled again to 0°C. Ethyl bromide (1.09g) is then added rapidly to the mixture and the reaction allowed to come to room

temperature for 2 hours, before evaporation of the solvent.

The residue is acidified with ice cold 10% hydrochloric acid and the product extracted twice with ether. The combined extracts are washed with dilute hydrochloric acid, water, then dried ($MgSO_4$) and evaporated to give 4-(4-chlorophenyl)-2-(2-phenylethyl)-2-ethylbutyric acid which was reduced without further purification to the title compound using the procedure of Part A of Preparation 3.

B. Similarly, substituting into the procedure of Part A hereinabove, for ethyl bromide: n-butyl bromide, methyl iodide, or i-propyl bromide, one obtains the corresponding 2-n-butyl-, 2-methyl- and 2-i-propyl-4-(4-chlorophenyl)-2-(2-phenylethyl)-butan-1-ols.

PREPARATION 5

Homologation of 4-Chlorophenyl-2-(2-phenylethyl)-n-butyl methanesulfonate

A. 4-Chlorophenyl-2-(2-phenylethyl)-n-butyl methanesulfonate (2.3g) (See Example 1, Part A) in a few ml of dimethyl formamide (DMF) is added to a cooled stirred suspension of anhydrous sodium cyanide (0.46g) in 5 ml of dry DMF. The mixture is heated overnight at 80°C poured into water (100 ml) and the product extracted with ether. The combined extracts are washed well with water, dried ($MgSO_4$) and evaporated to give 5-(4-chlorophenyl)-3-(2-phenylethyl)pentane nitrile, used directly in the next step.

5-(4-Chlorophenyl)-3-(2-phenylethyl)pentane nitrile (1.72g) is heated under reflux overnight in 10 ml of 50% aqueous sulfuric acid. The cooled mixture is then poured into water (100 ml) and extracted twice with ether. The ethereal extracts are washed with water, dried ($MgSO_4$) and evaporated to give 5-(4-chlorophenyl)-3-(2-phenylethyl)

pentanoic acid, which is then reduced according to the procedure of Preparation 3, Part A (2) to give 5-(4-chlorophenyl)-3-(2-phenylethyl)-n-pentan-1-ol.

B. Similarily, using the procedure of Part A of this Preparation, but substituting for 4-chlorophenyl-2-(2-phenylethyl)-n-butyl methanesulfonate the methanesulfonates of the alcohols listed in Preparation 3, Part B, one obtains:

1. 5-phenyl-3-(3-(4-methylphenyl)propyl)pentan-1-ol;
2. 6-(2-chlorophenyl)-3-(2-(4-methoxyphenyl)ethyl-hexan-1-ol;
3. 6-(2,4-dichlorophenyl)-3-(3-(3-chlorophenyl)-propyl)hexan-1-ol;
4. 4-(4-fluorophenyl)-3-(4-fluorobenzyl)butan-1-ol;
5. 5-(4-methylphenyl)-3-(1-(2,4-dimethylphenyl)-ethyl)pentan-1-ol;
6. 5-(4-fluorophenyl)-3-(2-(4-methoxyphenyl)-2-methylbutyl)pentan-1-ol;
7. 6-(3-chlorophenyl)-3-(1-(2-chlorobenzyl)-1-methylpropyl)hexan-1-ol.

## EXAMPLE 1

## 1-[4-(4-chlorophenyl)-2-(2-phenylethyl)-n-butyl]imidazole

A. 1-[4-(4-chlorophenyl)-2-(2-phenylethyl)-butan-1-ol in 10 ml of dry pyridine at 0° was treated dropwise with stirring with 0.87 ml of methanesulfonyl chloride, and stirring continued for a further hour at 0°C, and two hours at room temperature. The resulting mixture was poured into 100 ml of cold dilute hydrochloric acid and extracted with ethyl acetate (150 ml total). The extracts were washed with aqueous potassium carbonate solution, dried ($MgSO_4$) and evaporated to give 2.54 g of

4-(4-chlorophenyl)-2-(2-phenylethyl)-n-butyl methanesulfonate, used directly in the following:

A mixture of the above crude mesylate (2.54 g) and imidazole (3.4 g) in 10 ml of dry DMF was stirred at 145°C under nitrogen overnight. The solution was then poured into 250 ml of water, and the product extracted with ethyl acetate. The extracts were washed four times with water, dried ($MgSO_4$) and evaporated. The residue was chromatographed on silica gel, eluting with 10% methanol in dichloromethane to give 1.53 g of pure title compound as an oil.

The above free base was dissolved in ether and treated dropwise with nitric acid (d = 1.42) until precipitation was complete. The precipitate was collected and recrystallized from ethyl acetate to give 1.17 g of 1-[4-(4-chlorophenyl)-2-(2-phenylethyl)-n-butyl]imidazole nitrate as a white solid, m.p. 95-97°C.

B. Similarly, substituting into the procedure of Part A hereinabove, for 4-(4-chlorophenyl)-2-(2-phenylethyl)-butan-1-ol, the alcohols listed in part B of Preparation 3; one obtains:

1. 1-[4-phenyl-2-(3-(4-methylphenyl)propyl)-n-butyl]-imidazole;
2. 1-[5-(2-chlorophenyl)-2-(2-(4-methoxyphenyl)-ethyl)-n-pentyl]imidazole;
3. 1-[5-(2,4-dichlorophenyl)-2-(3-(3-chlorophenyl)-propyl)-n-pentyl]imidazole;
4. 1-[3-(4-fluorophenyl)-2-(4-fluorobenzyl)-n-propyl]-imidazole;
5. 1-[4-(4-methylphenyl)-2-(1-(2,4-dimethylphenyl)-ethyl)-n-butyl]imidazole;
6. 1-[4-(4-fluorophenyl)-2-(2-(4-methoxyphenyl)-2-methyl-butyl)-n-butyl]imidazole;

7. 1-[5-(3-chlorophenyl)-2-(1-(2-chlorobenzyl)-1-methyl-propyl)-n-pentyl]imidazole.

C. Similarly, substituting into the procedure of Part A hereinabove, for 4-(4-chlorophenyl)-2-(2-phenylethyl)-butan-1-ol,

2-ethyl-4-(4-chlorophenyl)-2-(2-phenylethyl)-butan-1-ol;

2-n-butyl-4-(4-chlorophenyl)-2-(2-phenylethyl)-butan-1-ol;

2-methyl-4-(4-chlorophenyl)-2-(2-phenylethyl)-butan-1-ol; and

2-i-propyl-4-(4-chlorophenyl)-2-(2-phenylethyl)-butan-1-ol;

one obtains the corresponding:

1-[2-ethyl-4-(4-chlorophenyl)-2-(2-phenylethyl)-n-butyl]imidazole;

1-[2-n-butyl-4-(4-chlorophenyl)-2-(2-phenylethyl)-n-butyl]imidazole;

1-[2-methyl-4-(4-chlorophenyl)-2-(2-phenylethyl)-n-butyl]imidazole; and

1-[2-i-propyl-4-(4-chlorophenyl)-2-(2-phenylethyl)-n-butyl]imidazole.

## EXAMPLE 2

### Conversion of 1-[5-(4-methylphenyl)-3-oxo-n-pentyl]imidazole to 1-[5-(4-methylphenyl-3-benzyl-n-pentyl]imidazole

A. 2.3 g 1-[5-(4-methylphenyl)-3-oxo-n-pentyl]-imidazole is dissolved in 20 ml dry THF, and added dropwise with stirring under nitrogen to an ice cooled solution of benzylmagnesium chloride [from benzyl chloride (1.58g) and magnesium (0.30g] in 15 ml of tetrahydrofuran. The mixture is allowed to come to room temperature overnight and quenched by the addition of

saturated aqueous ammonium chloride solution. After addition of water, the product is extracted twice with ethyl acetate and the combined extracts washed, dried ($MgSO_4$) and evaporated to give 1-[5-(4-methylphenyl)-3-benzyl-3-hydroxy-n-pentyl]imidazole. If necessary, purification is effected by chromatography on silica gel, eluting with 5% methanol in methylenechloride.

B. 1-[5-(4-methylphenyl)-3-benzyl-3-hydroxy-n-pentyl] imidazole (1.5g) in 15 ml of pyridine is treated at 0°C with stirring with 3 ml of thionyl chloride. The mixture is stirred for a further four hours at 0°C and allowed to come to room temperature overnight. The pyridine was evaporated in vacuo and the residue treated with dilute aqueous potassium carbonate and extracted with ether. The ether extracts were washed with water, dried ($MgSO_4$) and evaporated to give 1-[3-[2-(4-methylphenyl)ethyl]-4-phenylprop-3-enyl]imidazole and its isomers.

C. A solution of 1-[3-[2-(4-methylphenyl)ethyl]-4-phenylprop-3-enyl]imidazole (and isomers) (1.0g) in ethanol (20 ml) is hydrogenated at ambient pressure and temperature over a 5%-palladium on charcoal catalyst (100 mg). (It may be necessary to pretreat with the catalyst to remove catalyst poisons.) After the uptake of hydrogen ceases, the solution is filtered and evaporated to dryness to give 1-[5-(4-methylphenyl)-3-benzyl-n-pentyl]imidazole as an oil.

This oil is taken up in ether and treated dropwise with oxalic acid in ether until precipitation is complete. The solid is collected and recrystalized from ethyl acetate to give the oxalate salt.

D. Alternative Procedure

A solution of diethyl 2,4-dimethylbenzylphosphonate (3.07g) in 50 ml of dry tetrahydrofuran at -78°C is treated with stirring under nitrogen with 1.6 m n-butyllithium in hexane (7.25 ml). After ten minutes at this temperature, 1-[4-phenyl-2-oxo-n-butyl]imidazole. (1.71 g) in 10 ml of tetrahydrofuran is added dropwise and the mixture allowed to come slowly to room temperature overnight. After an additional six hours, under reflux, the solvent was evaporated, water added, and the product extracted three times with ethyl acetate. The combined extracts are washed, dried ($MgSO_4$) and evaporated, and the residue chromatographed on silica gel eluting with 6% methanol in methylene chloride to give 1-[2-(2-phenylethyl)-3-(2,4-dimethylphenyl)prop-2-enyl]imidazole.

This is hydrogenated as in paragragh C hereinabove to give 1-[4-phenyl-2-(2,4-dimethylbenzyl)-n-butyl] imidazole.

E. Similarily, substituting into the procedure of Part A, B, C or D hereinabove, for 1-[5-(4-chlorophenyl)-3-oxo-n-pentyl]-imidazole,

1-[5-(2,4-dichlorophenyl)-3-oxo-n-pentyl]imidazole;
1-[5-(4-fluorophenyl)-3-oxo-n-pentyl]imidazole;
1-[5-(3-chlorophenyl)-3-oxo-n-pentyl]imidazole;
1-[6-(4-dichlorophenyl)-4-oxo-n-hexyl]imidazole;
1-[6-(2,4-dichlorophenyl)-4-oxo-n-hexyl]imidazole;
1-[6-(4-fluorophenyl)-4-oxo-n-hexyl]imidazole;
1-[6-(3-chlorophenyl)-4-oxo-n-hexyl]imidazole; and

one obtains respectively,

1-[5-(2,4-dichlorophenyl)-3-(3-(4-methylphenyl)-propyl)-n-pentyl]imidazole;

1-[5-(4-fluorophenyl)-3-(3-(4-methylphenyl)propyl)-n-pentyl]imidazole;

1-[5-(3-chlorophenyl)-3-(3-(4-methylphenyl)propyl)-n-pentyl]imidazole;

1-[6-(4-chlorophenyl)-4-(3-(4-methylphenyl)propyl)-n-hexyl]imidazole;

1-[6-(2,4-dichlorophenyl)-4-(3-(4-methylphenyl)-propyl)-n-hexyl]imidazole;

1-[6-(4-fluorophenyl)-4-(3-(4-methylphenyl)propyl)-n-hexyl]imidazole; and

1-[6-(3-chlorophenyl)-4-(3-(4-methylphenyl)propyl)-n-hexyl]imidazole.

Following the procedures outlined in Example 1 or 2 herein, the following compounds are prepared:

1-[4-phenyl-2-benzyl-n-butyl]imidazole;

1-[4-phenyl-2-(2-chlorobenzyl)-n-butyl]imidazole;

1-[4-phenyl-2-(4-chlorobenzyl)-n-butyl]imidazole;

1-[4-phenyl-2-(2-methylbenzyl)-n-butyl]imidazole;

1-[4-phenyl-2-(4-methylbenzyl)-n-butyl]imidazole;

1-[4-phenyl-2-(2-phenylethyl)-n-butyl]imidazole;

1-[4-phenyl-2-(2-(2-chlorophenyl)ethyl)-n-butyl]imidazole;

1-[4-phenyl-2-(2-(4-chlorophenyl)ethyl)-n-butyl]imidazole;

1-[4-phenyl-2-(2-(2-methylphenyl)ethyl)-n-butyl]imidazole;

1-[4-phenyl-2-(2-(4-methylphenyl)ethyl)-n-butyl]imidazole;

1-[4-(4-chlorophenyl)-2-benzyl-n-butyl]imidazole;

1-[4-(4-chlorophenyl)-2-(2-chlorobenzyl)-n-butyl]imidazole, m.p. 112-113.5°C as nitrate;

1-[4-(4-chlorophenyl)-2-(4-fluorobenzyl)-n-butyl]-imidazole, m.p. 123-124.5°C as nitrate;

1-[4-(4-chlorophenyl)-2-(4-chlorobenzyl)-n-butyl]imidazole, m.p. 123-125.5°C as nitrates;

1-[4-(4-chlorophenyl)-2-(2-methylbenzyl)-n-butyl]imidazole;

1-[4-(4-chlorophenyl)-2-(4-methylbenzyl)-n-butyl]imidazole;

1-[4-(4-chlorophenyl)-2-(2-phenylethyl)-n-butyl]imidazole;

1-[4-(4-chlorophenyl)-2-(2-(2-chlorophenyl)ethyl)-n-butyl]imidazole, m.p. 130.5-133°C as nitrate;

1-[4-(4-chlorophenyl)-2-(2-(4-chlorophenyl)ethyl)-n-butyl]imidazole, m.p. 132.5-135°C as nitrate;

1-[4-(4-chlorophenyl)-2-(2-(2-methylphenyl)ethyl)-n-butyl]imidazole;

1-[4-(4-chlorophenyl)-2-(2-(4-methylphenyl)ethyl)-n-butyl]imidazole;

1-[4-(4-chlorophenyl)-2-(2,4-dichlorobenzyl)-n-butyl]-imidazole, m.p. 119.5-122°C as nitrate;

1-[4-(4-chlorophenyl)-2-(3,4-dichlorobenzyl)-n-butyl]-imidazole, m.p. 133-134.5°C as nitrate;

1-[4-(4-methylphenyl)-2-benzyl-n-butyl]imidazole;

1-[4-(4-methylphenyl)-2-(2-chlorobenzyl)-n-butyl]imidazole;

1-[4-(4-methylphenyl)-2-(4-chlorobenzyl)-n-butyl]imidazole;

1-[4-(4-methylphenyl)-2-(2-methylbenzyl)-n-butyl]imidazole;

1-[4-(4-methylphenyl)-2-(4-methylbenzyl)-n-butyl]imidazole;

1-[4-(4-methylphenyl)-2-(2-phenylethyl)-n-butyl]imidazole;

1-[4-(4-methylphenyl)-2-(2-(2-chlorophenyl)ethyl)-n-butyl]imidazole;

1-[4-(4-methylphenyl)-2-(2-(4-chlorophenyl)ethyl)-n-butyl]imidazole;

1-[4-(4-methylphenyl)-2-(2-(2-methylphenyl)ethyl)-n-butyl]imidazole;

1-[4-(4-methylphenyl)-2-(2-(4-methylphenyl)ethyl)-n-butyl]imidazole;

1[4-(4-methoxyphenyl)-2-benzyl-n-butyl]imidazole;

1[4-(4-methoxyphenyl)-2-(2-chlorobenzyl)-n-butyl]imidazole;

1[4-(4-methoxyphenyl)-2-(4-chlorobenzyl)-n-butyl]imidazole;

1[4-(4-methoxyphenyl)-2-(2-methylbenzyl)-n-butyl]imidazole;

1[4-(4-methoxyphenyl)-2-(4-methylbenzyl)-n-butyl]imidazole;

1[4-(4-methoxyphenyl)-2-(2-phenylethyl)-n-butyl]imidazole;

1[4-(4-methoxyphenyl)-2-(2-(2-chlorophenyl)ethyl)-n-butyl]imidazole;

1[4-(4-methoxyphenyl)-2-(2-(4-chlorophenyl)ethyl)-n-butyl]imidazole;

1[4-(4-methoxyphenyl)-2-(2-(2-methylphenyl)ethyl)-n-butyl]imidazole;

1[4-(4-methoxyphenyl)-2-(2-(4-methylphenyl)ethyl)-n-butyl]imidazole;

1-[4-(2,4-dichlorophenyl)-2-benzyl-n-butyl]imidazole;

1-[4-(2,4-dichlorophenyl)-2-(2-chlorobenzyl)-n-butyl]imidazole;

1-[4-(2,4-dichlorophenyl)-2-(4-chlorobenzyl)-n-butyl]imidazole;

1-[4-(2,4-dichlorophenyl)-2-(2-methylbenzyl)-n-butyl]imidazole;

1-[4-(2,4-dichlorophenyl)-2-(4-methylbenzyl)-n-butyl]imidazole;

1-[4-(2,4-dichlorophenyl)-2-(2-phenylethyl)-n-butyl]imidazole;

1-[4-(2,4-dichlorophenyl)-2-(2-(2-chlorophenyl)ethyl)-n-butyl]imidazole;

1-[4-(2,4-dichlorophenyl)-2-(2-(4-chlorophenyl)ethyl)-n-butyl]imidazole;

1-[4-(2,4-dichlorophenyl)-2-(2-(2-methylphenyl)ethyl)-n-butyl]imidazole;

1-[4-(2,4-dichlorophenyl)-2-(2-(4-methylphenyl)ethyl)-n-butyl]imidazole;

1-[4-(2,4-dimethylphenyl)-2-benzyl-n-butyl]imidazole;

1-[4-(2,4-dimethylphenyl)-2-(2-chlorobenzyl)-n-butyl]imidazole;

1-[4-(2,4-dimethylphenyl)-2-(4-chlorobenzyl)-n-butyl]imidazole;

1-[4-(2,4-dimethylphenyl)-2-(2-methylbenzyl)-n-butyl]imidazole;

1-[4-(2,4-dimethylphenyl)-2-(4-methylbenzyl)-n-butyl]imidazole;

1-[4-(2,4-dimethylphenyl)-2-(2-phenylethyl)-n-butyl]imidazole;

1-[4-(2,4-dimethylphenyl)-2-(2-(2-chlorophenyl)ethyl)-n-butyl]imidazole;

1-[4-(2,4-dimethylphenyl)-2-(2-(4-chlorophenyl)ethyl)-n-butyl]imidazole;

1-[4-(2,4-dimethylphenyl)-2-(2-(2-methylphenyl)ethyl)-n-butyl]imidazole;

1-[4-(2,4-dimethylphenyl)-2-(2-(4-methylphenyl)ethyl)-n-butyl]imidazole;

1-[4-(4-chlorophenyl)-2-(1-phenylethyl)-n-butyl]imidazole;

1-[4-phenyl-2-(1-phenylethyl)-n-butyl]imidazole;

1-[4-(4-chlorophenyl)-2-(1-(2-chlorophenyl)ethyl)-n-butyl]imidazole;

1-[4-phenyl-2-(1-(2-chlorophenyl)ethyl)-n-butyl]imidazole;

1-[4-(4-chlorophenyl)-2-(1-(4-chlorophenyl)ethyl)-n-butyl]imidazole;

1-[4-phenyl-2-(1-(4-chlorophenyl)ethyl)-n-butyl]imidazole;

1-[4-(4-chlorophenyl)-2-(1-(2-methylphenyl)ethyl)-n-butyl]imidazole;

1-[4-phenyl-2-(1-(2-methylphenyl)ethyl)-n-butyl]imidazole;

1-[4-(4-chlorophenyl)-2-(1-(4-methylphenyl)ethyl)-n-butyl]imidazole;

1-[4-phenyl-2-(1-(4-methylphenyl)ethyl)-n-butyl]imidazole;

1-[4-(4-chlorophenyl)-2-(1-phenyl-1-methylethyl)-n-butyl]imidazole;

1-[4-phenyl-2-(1-phenyl-1-methylethyl)-n-butyl]imidazole;

1-[4-(4-chlorophenyl)-2-methyl-2-(2-(2-chlorophenyl)-ethyl-n-butyl]imidazole;

1-[4-(4-chlorophenyl)-2-methyl-2-(2-phenylethyl)-n-butyl]imidazole;

1-[4-(4-chlorophenyl)-2-methyl-2-(2-chlorobenzyl)-n-butyl]imidazole;

1-[4-(4-chlorophenyl)-2-methyl-2-benzyl-n-butyl]imidazole;

1-[4-(4-chlorophenyl)-2-methyl-2-(4-chlorobenzyl)-n-butyl]imidazole;

1-[4-phenyl-2-methyl-2-(2-(2-chlorophenyl)ethyl)-n-butyl]imidazole;

1-[4-phenyl-2-methyl-2-(2-phenylethyl-n-butyl]imidazole;

1-[4-phenyl-2-methyl-2-(2-chlorobenzyl)-n-butyl]imidazole;

1-[4-phenyl-2-methyl-2-benzyl-n-butyl]imidazole;

1-[4-phenyl-2-methyl-2-(4-chlorobenzyl)-n-butyl]imidazole;

1-[4-phenyl-2-n-butyl-2-(2-phenylethyl)-n-butyl]imidazole;

1-[4-phenyl-2-n-butyl-2-(2-(4-fluorophenyl)ethyl)-n-butyl]imidazole;

1-[4-(4-chlorophenyl)-2-n-butyl-2-(2-(4-methoxyphenyl)ethyl)-n-butyl]imidazole;

1-[5-phenyl-2-benzyl-n-pentyl]imidazole;

1-[5-phenyl-2-(2-chlorobenzyl)-n-pentyl]imidazole;

1-[5-phenyl-2-(4-chlorobenzyl)-n-pentyl]imidazole;

1-[5-phenyl-2-(2-methylbenzyl)-n-pentyl]imidazole;

1-[5-phenyl-2-(2-phenylethyl)-n-pentyl]imidazole;

1-[5-phenyl-2-(2-(2-chlorophenyl)ethyl)-n-pentyl]imidazole;

1-[5-(4-chlorophenyl)-2-benzyl-n-pentyl]imidazole;

1-[5-(4-chlorophenyl)-2-(2-chlorobenzyl)-n-pentyl]imidazole;

1-[5-(4-chlorophenyl)-2-(4-chlorobenzyl)-n-pentyl]imidazole;

1-[5-(4-chlorophenyl)-2-(2-methylbenzyl)-n-pentyl]imidazole;

1-[5-(4-chlorophenyl)-2-(2-phenylethyl)-n-pentyl]imidazole;

1-[5-(4-chlorophenyl)-2-(2(-2-chlorophenyl)ethyl)-n-pentyl]imidazole;

1-[5-phenyl-2-methyl-2-(3-phenylpropyl)-n-pentyl]imidazole;

1-[5-(4-fluorophenyl)-2-methyl-2-(3-(4-fluorophenyl)propyl)-n-pentyl]imidazole;

1-[3-phenyl-2-benzyl-n-propyl]imidazole;

1-[3-phenyl-2-(2-chlorobenzyl)-n-propyl]imidazole;

1-[3-(4-chlorophenyl)-2-benzyl-n-propyl]imidazole;

1-[3-(4-chlorophenyl)-2-(2-chlorobenzyl)-n-propyl]imidazole;

1-[3-(2-chlorophenyl)-2-benzyl-n-propyl]imidazole;

1-[3-(2-chlorophenyl)-2-(2-chlorobenzyl)-n-propyl]imidazole;

1-[3-(4-methylphenyl)-2-benzyl-n-propyl]imidazole;

1-[3-(4-methylphenyl)-2-(2-chlorobenzyl)-n-propyl]imidazole;

1-[3-(4-methoxyphenyl)-2-benzyl-n-propyl]imidazole;

1-[3-(4-methoxyphenyl)-2-(2-chlorobenzyl)-n-propyl]imidazole;

1-[3-(4-fluorophenyl)-2-benzyl-n-propyl]imidazole;

1-[3-(4-fluorophenyl)-2-(2-chlorobenzyl)-n-propyl]imidazole;

1-[3-(4-chlorophenyl)-2-(4-chlorobenzyl)-n-propyl]imidazole;

1-[3-(4-chlorophenyl)-2-methyl-2-(4-chlorobenzyl)-n-propyl]imidazole;

1-[3-(4-phenyl-2-n-butyl-2-benzyl-n-propyl]imidazole;

1-[5-phenyl-3-benzyl-n-pentyl]imidazole;

1-[5-phenyl-3-(2-chlorobenzyl)-n-pentyl]imidazole;

1-[5-phenyl-3-(4-chlorobenzyl)-n-pentyl]imidazole;

1-[5-phenyl-3-(2-phenylethyl)-n-pentyl]imidazole;

1-[5-phenyl-3-(2-(2-chlorophenyl)ethyl)-n-pentyl]imidazole;

1-[5-phenyl-3-(2-(4-chlorophenyl)ethyl)-n-pentyl]imidazole;

1-[5-(4-chlorophenyl)-3-benzyl-n-pentyl]imidazole;

1-[5-(4-chlorophenyl)-3-(2-chlorobenzyl)-n-pentyl]imidazole;

1-[5-(4-chlorophenyl)-3-(4-chlorobenzyl)-n-pentyl]imidazole;

1-[5-(4-chlorophenyl)-3-(2-phenylethyl)-n-pentyl]imidazole;

1-[5-(4-chlorophenyl)-3-(2-(2-chlorophenyl)ethyl)-n-pentyl]imidazole;

1-[5-(4-chlorophenyl)-3-(2-(4-chlorophenyl)ethyl)-n-pentyl]imidazole;

1-[5-(2-chlorophenyl)-3-benzyl-n-pentyl]imidazole;

1-[5-(2-chlorophenyl)-3-(2-chlorobenzyl)-n-pentyl]imidazole;

1-[5-(2-chlorophenyl)-3-(4-chlorobenzyl)-n-pentyl]imidazole;

1-[5-(2-chlorophenyl)-3-(2-phenylethyl)-n-pentyl]imidazole;

1-[5-(2-chlorophenyl)-3-(2-(2-chlorophenyl)ethyl)-n-pentyl]imidazole;

1-[5-(2-chlorophenyl)-3-(2-(4-chlorophenyl)ethyl)-n-pentyl]imidazole;

1-[5-phenyl-3-methyl-3-(2-phenylethyl)-n-pentyl]-imidazole;

1-[5-phenyl-3-methyl-3-(2-(2-chlorophenyl)ethyl)-n-pentyl]imidazole;

1-[5-(4-chlorophenyl)-3-methyl-3-(2-phenylethyl)-n-pentyl]imidazole;

1-[5-(4-chlorophenyl)-3-methyl-3-(2-(2-chlorophenyl)-ethyl)-n-pentyl]imidazole;

1-[6-phenyl-3-benzyl-n-hexyl]imidazole;

1-[6-phenyl-3-(2-phenylethyl)-n-hexyl]imidazole;

1-[6-phenyl-3-(2-chlorobenzyl)-n-hexyl]imidazole;

1-[6-(4-chlorophenyl)-3-(2-phenylethyl)-n-hexyl]imidazole;

1-[6-(4-chlorophenyl)-3-(2-(4-chlorophenyl)ethyl)n-hexyl]imidazole;

1-[7-(4-chlorophenyl)-4-(2-chlorobenzyl)-n-heptyl]imidazole;

1-[7-(4-chlorophenyl)-4-(4-chlorobenzyl)-n-heptyl]imidazole;

1-[7-(4-chlorophenyl)-4-(2-phenylethyl)-n-heptyl]imidazole;

1-[7-(4-chlorophenyl)-4-(2-(2-chlorophenyl)ethyl)-n-heptyl]imidazole.

EXAMPLE 3

A. 1.0g of 1-[4-(4-chlorophenyl)-2-(2-phenylethyl)-n-butyl]imidazole is dissolved in ether, and nitric acid added dropwise until precipitation is complete. The resulting nitrate is then collected and recrystallized from ethyl acetate.

B. In a similar manner, all compounds of Formula I in free base form may be converted to the acid addition salts by treatment with the appropriate acid, for example, hydrochloric acid, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

EXAMPLE 4

Conversion of Salt to Free Base

1.0 g of 1-[4-(4-chlorophenyl)-2-(2-phenylethyl)-n-butyl]imidazole.HCl suspended in 50 ml of ether is stirred with excess dilute aqueous potassium carbonate solution until the salt is completely dissolved. The organic layer is then separated, washed twice with water, dried over magnesium sulfate and evaporated to yield 1-[4-(4-chlorophenyl)-2-(2-phenylethyl)-n-butyl]imidazole as the free base.

EXAMPLE 5

Direct interchange of acid addition salts

1-[4-(4-chlorophenyl)-2-(2-phenylethyl)-n-butyl] imidazole acetate (1.0 g) is dissolved in 50 ml 50% aqueous sulfuric acid, and the solution evaporated to dryness. The product is suspended in ethanol and

filtered, air dried and the resulting 1-[4-(4-chlorophenyl)-2-(2-phenylethyl)-n-butyl]imidazole.$H_2SO_4$ is crystallized from methanol/acetone.

The active ingredient in Examples 6-7 is 1-[2-(2-phenylethyl)-4-(4-chlorophenyl)butyl]imidazole as the nitrate salt; however any compound of the invention may be utilized.

EXAMPLE 6

The following illustrates formulations for vaginal administration in contraceptive uses of the compounds.

a) Water soluble vaginal cream

| Ingredients | % w/w |
|---|---|
| Active ingredient | 1.0 |
| Cetostearyl alcohol | 12.0 |
| Polysorbate 60 | 2.0 |
| Sorbitan monostearate | 2.0 |
| Mineral oil | 2.0 |
| Propylene glycol | 4.0 |
| Benzyl alcohol | 1.0 |
| Butylated hydroxyanisole | 0.01 |
| Purified water qs ad | 100.0 |

All ingredients except the active ingredient, water, and 10% of the Polysorbate 60 are mixed and heated to 70-80°. 85% of the required water is separately heated to 70-80°. The remaining Polysorbate 60 and 10% of the water are dissolved together at 50-60°, and the active ingredient dissolved therein.

The heated water is then added to the heated emulsifying ingredients using a homomixer at medium speed, with a final increase to high speed for 1 minute after mixing is complete. The homomixer is removed and mixing continued gently until the mixture congeals and cools to room temperature; whereupon the active

ingredient, previously dissolved as described above, is added, and gentle mixing continued for 20-30 minutes. The remaining 5% of water is then used to rinse the vessels containing the premixed active ingredient and the rinse added to the total mixture. Mixing is continued and further water added if necessary.

For each application approximately 1 gm of the cream is vaginally administered to an adult human female with a suitable syringe.

b) Vaginal jelly

| Ingredients | % w/w |
|---|---|
| Active ingredient | 1.00 |
| Tragacanth | 3.00 |
| Acacia | 0.53 |
| Glycerin | 5.00 |
| Boric acid | 3.00 |
| Ricinoleic acid | 0.75 |
| p-hydroxybenzoic acid, propyl ester | 0.05 |
| Purified water qs ad | 100.0 |

The tragacanth and acacia are intermixed thoroughly with the glycerin; ricinoleic acid and active ingredient are then added to the mixture. The p-hydroxybenzoate and boric acid are dissolved in water (with heating if necessary), and the solution is added to the prior mixture, with stirring and warming to dissolve. The mixture becomes gelatinous upon cooling.

For each application approximately 1 gram of the gel is vaginally administered to an adult human female with a suitable syringe.

c) Vaginal suppository

| Ingredients | % w/w |
|---|---|
| Active ingredient | 1.0 |
| Polyethylene glycol 4000 | 20.0 |
| Butylated hydroxyanisole | 0.01 |

| | |
|---|---|
| Polyethylene glycol 1000 qs ad | 100.0 |

The polyethylene glycol solids are mixed and heated to 70-80°, and the BHA dissolved in the mixture. After cooling to 45°C, the active ingredient is suspended in the above mixture by stirring. The suspension is poured into molds which are of sufficient size to form suppositories of about 3 gm each, and cooled.

d) Effervescent vaginal tablets

| Ingredients | % w/w |
|---|---|
| Active ingredient | 1.0 |
| Anhydrous citric acid | 35.0 |
| Sodium bicarbonate | 15.0 |
| Polyethylene glycol 6000 | 20.0 |
| Lactose qs ad | 100.0 |

The above ingredients are combined and granulated using methanol as the solvent. The formulation is then dried and formed into tablets containing 20 mg of active compound with an appropriate tableting medicine.

e) Vaginal spray-foam

| Ingredients | % w/w |
|---|---|
| Active ingredient | 2.0 |
| Emulsion base | 90.0 |
| Propellant 12/114 (40:60) | 8.0 |

The emulsion base is made up according to the following % w/w composition:

| | |
|---|---|
| myristic acid | 1.33 |
| stearic acid | 5.33 |
| cetyl alcohol | 0.50 |
| lanolin | 0.20 |
| isopropyl myristate | 1.33 |
| triethanolamine | 3.33 |
| glycerin | 4.70 |
| polyvinylpyrrolidine | 0.34 |

| | |
|---|---|
| purified water | 82.93 |

The ingredients of the emulsion base, except for the water, are mixed in a stainless steel container kept at 70-80°. 80% of the water to be used is also heated to 70-80° and mixed during heating with a homomixer at moderate speed. After complete addition, the speed of mixing is increased for several minutes. The mixture is then cooled to room temperature and a solution containing the active ingredient in the remaining 20% water is added with continuous mixing. The preparation is placed in an appropriate spray can, topped with the propellant mixture, and sealed.

For each application approximately 0.5 gm of the foam are vaginally administered.

f) Vaginal soluble waffle

| Ingredients | % w/w |
|---|---|
| Active ingredient | 1.0 |
| Starch | 10.0 |
| Water soluble lanolin qs ad | 100.0 |

The above ingredients are thoroughly mixed and pressed into 0.8 gm waffles with a suitable press.

EXAMPLE 7

The following pharmaceutical composition is representative of those which may be used for oral administration to a male mammal for contraceptive use.

| Ingredients | Parts by Weight |
|---|---|
| Active ingredient | 200 |
| Magnesium stearate | 3 |
| Starch | 30 |
| Lactose | 116 |
| Polyvinylpyrrolidone | 3 |

The above ingredients are combined and granulated using methanol as the solvent. The formulation is then dried

and formed into tablets containing 200 milligrams of active compound with an appropriate tableting machine.

EXAMPLE 8

Biodata

Assay for Spermicidal Activity

The entire ejaculate, including both sperm-rich and prostatic secretion fractions, is collected from a male Beagle (KAR-2) into a prewarmed collection tube. Immediately after collection the tube is placed into a 37°C water bath. Within 15 minutes of collection the semen sample mixed by repeated inversion is aliquoted into prewarmed tubes and a solution of compound to be tested added in a ratio of 1:3 test solutions: semen. Identical vehicle control(s) are always assayed concomitantly. As soon as possible after addition of test solution (time 0) a drop of semen is removed, placed on a prewarmed slide and examined microscopically for sperm motility, graded active to inactive on a scale of 5 to 0. These measures of motility are then repeated at specified time intervals.

| Compound | Final Concentration tested (μg/ml) | time of assay (mm) | Motility* |
|---|---|---|---|
| 1. | 2.5 | 0<br>30<br>60 | 4<br>0<br>0 |
| 2. | 2.5 | 0<br>30<br>60 | 4<br>0<br>0 |
| 3. | 2.5 | 0<br>30<br>60 | 4<br>0<br>0 |
| 4. | 2.5 | 0<br>30<br>60 | 4<br>0<br>0 |
| 5. | 2.5 | 0<br>30<br>60 | 4<br>1①<br><1② |
| 6. | 2.5 | 0<br>30<br>60 | 4<br><1②<br><1③ |
| 7. | 2.5 | 0<br>30<br>60 | 4<br><1<br>0 |
| 8. | 2.5 | 0<br>30<br>60 | 4<br><1②<br><1① |
| 0.9%NaCl+ DMSO | — | 0<br>30<br>60 | 4<br>4<br>4 |

* Motility is listed as active to inactive on a scale of 5 to 0.

① Most appeared non-motile, a few with good forward motility.

② very few seen motile.

③ same as ②, but fewer motile.

Compounds

1. 1-[4-(4-chlorophenyl)-2-(2-phenylethyl)-n-butyl]imidazole nitrate;

2. 1-[4-(4-chlorophenyl)-2-(4-fluorobenzyl)-n-butyl]imidazole nitrate;

3. 1-[4-(4-chlorophenyl)-2-(2-chlorobenzyl)-n-butyl]imidazole nitrate;

4. 1-[4-(4-chlorophenyl)-2-(2-(2-chlorophenyl)-ethyl)-n-butyl]imidazole nitrate;

5. 1-[4-(4-chlorophenyl)-2-(2-(4-chlorophenyl)-ethyl)-n-butyl]imidazole nitrate;

6. 1-[4-(4-chlorophenyl)-2-(4-chlorobenzyl)-n-butyl]imidazole nitrate;

7. 1-[4-(4-chlorophenyl)-2-(2,4-dichlorobenzyl)-n-butyl]imidazole nitrate;

8. 1-[4-(4-chlorophenyl)-2-(3,4-dichlorobenzyl)-n-butyl]imidazole nitrate;

WHAT IS CLAIMED IS:

1. A compound of the formula

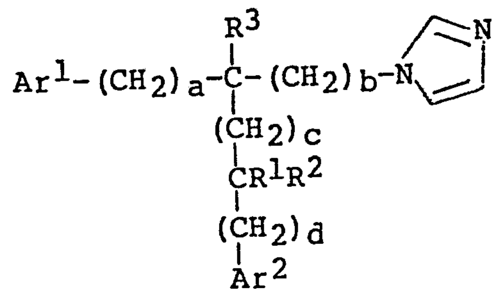

(I)

wherein:

$Ar^1$ and $Ar^2$ are each independently optionally substituted phenyl where substituted means that 1, 2, 3, 4 and/or 5 hydrogens on the phenyl ring are replaced by halo, alkyl, alkoxy, or trifluoromethyl;

$R^1$, $R^2$ and $R^3$ are each independently hydrogen or alkyl where alkyl is a branched or unbranched hydrocarbon residue containing 1, 2, 3 or 4 carbons;

a is an integer from 1-3;

b is an integer from 1-3;

c is an integer from 0-2; and

d is an integer from 0-2, such that (c + d) equals 0-2;

and the pharmaceutically acceptable non-toxic acid addition salts thereof.

2. The compound of Claim 1 and the pharmaceutically acceptable non-toxic acid addition salts thereof, wherein:

$Ar^1$ and $Ar^2$ are each independently selected from the group consisting of unsubstituted phenyl or phenyl substituted by substituents selected from the group consisting of halo, alkyl, and methoxy; and

$R^1$, $R^2$ and $R^3$ are each independently methyl or

hydrogen.

3. The compound of Claim 2 and the pharmaceutically acceptable non-toxic acid addition salts thereof, wherein:

$R^1$, $R^2$ and $R^3$ are all hydrogen;

$Ar^1$ and $Ar^2$ are each independently selected from the group consisting of unsubstituted phenyl or phenyl substituted with one to two identical substituents selected from the group consisting of fluoro, chloro, methyl, or methoxy;

b is 1 or 2,

c is 0 or 1, and

d is 0 or 1.

4. The compound of Claim 3 and the pharmaceutically acceptable acid addition salts thereof, wherein a is 2 or 3.

5. The compound of Claim 4 and the pharmaceutically acceptable non-toxic acid addition salts thereof, wherein:

$Ar^1$ is selected from the group consisting of phenyl, 4-chlorophenyl, 4-fluorophenyl, 4-methylphenyl, 4-methoxyphenyl;

$Ar^2$ is selected from the group consisting of phenyl, 2-chlorophenyl, 4-chlorophenyl, 3,4-dichlorophenyl, 2,4-dichlorophenyl, 2-methylphenyl, 4-methylphenyl, 2,4-dimethylphenyl, 2-methoxyphenyl, or 4-methoxyphenyl;

a is 2,

b is 1, and

(c + d) is 0 or 1.

6. The compound of Claim 5, which is 1-[2-(2-phenylethyl)-4-(4-chlorophenyl)-n-butyl]-imidazole, 1-[2-(2-(2-chlorophenyl)ethyl)-4-(4-chlorophenyl)-n-butyl] imidazole or 1-[2-(2-(4-chlorophenyl)ethyl)-4-(4-chlorophenyl)-n-butyl] imidazole, and the pharmaceutically acceptable non-toxic acid addition salts thereof.

7. The compound of Claim 5, which is 1-[2-(4-fluorobenzyl)-4-(4-chlorophenyl)-n-butyl] imidazole, 1-[2-(2-chlorobenzyl)-4-(4-chlorophenyl)-n-butyl] imidazole or 1-[2-(4-chlorobenzyl)-4-(4-chlorophenyl)-n-butyl]-imidazole, and the pharmaceutically acceptable non-toxic acid addition salts thereof.

8. The compound of Claim 5 which is 1-[4-(4-chlorophenyl)-2-(2,4-dichlorobenzyl)-n-butyl] imidazole or 1-[4-(4-chlorophenyl)-2-(3,4-dichlorobenzyl)-n-butyl] imidazole, and the pharmaceutically acceptable non-toxic acid addition salts thereof.

9. A pharmaceutical contraceptive composition, which comprises a spermicidally effective amount of the compound of Claim 1 or a pharmaceutically acceptable non-toxic acid addition salt thereof, in admixture with at least one pharmaceutically acceptable excipient.

10. The composition of Claim 9 wherein said composition is formulated for vaginal administration to the female.

11. The composition of Claim 9 wherein said composition is formulated for oral administration to the male.

12. A process for preparing a compound of the formula:

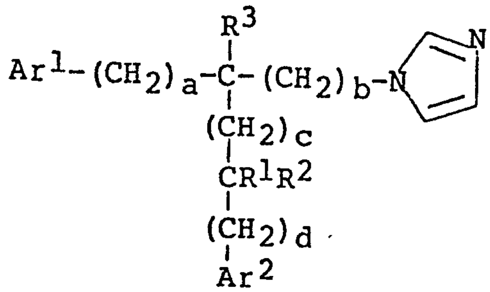

(I)

and the pharmaceutically acceptable non-toxic salts thereof, wherein:

$Ar^1$ and $Ar^2$ are each independently optionally substituted phenyl where substituted means that 1, 2, 3, 4 and/or 5 hydrogens on the phenyl ring are replaced by halo, alkyl, alkoxy, or trifluoromethyl;

$R^1$, $R^2$ and $R^3$ are each independently hydrogen or alkyl where alkyl is a branched or unbranched hydrocarbon residue containing 1, 2, 3 or 4 carbons;
alkyl;

a is an integer from 1-3;

b is an integer from 1-3;

c is an integer from 0-2; and

d is an integer from 0-2, such that (c + d) equals 0-2;

and the pharmaceutically acceptable non-toxic salts thereof which process comprises:

a) treating a compound of the formula

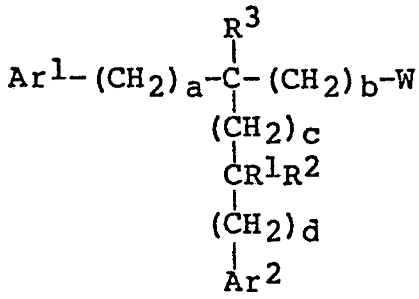

III

wherein:

$Ar^1$, $Ar^2$, $R^1$, $R^2$, $R^3$, a, b, c, d, are as defined herein and W is a leaving group, with imidazole; optionally followed by

b) converting the resulting free base to a pharmaceutically acceptable non-toxic salt.

c) converting a salt of the compound of Formula I to a free base; or

d) converting a salt of the compound of Formula I to another salt.

13. A process for preparing a compound of the formula

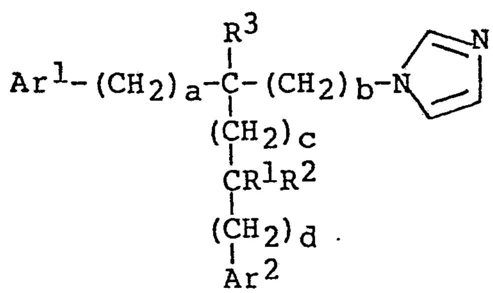

(I)

and the pharmaceutically acceptable non-toxic salts thereof; wherein:

$Ar^1$ and $Ar^2$ are each independently optionally substituted phenyl where substituted means that 1, 2, 3, 4 and/or 5 hydrogens on the phenyl ring are replaced by halo, alkyl, alkoxy, or trifluoromethyl;

$R^1$, $R^2$ and $R^3$ are each independently hydrogen or alkyl where alkyl is a branched or unbranched hydrocarbon residue containing 1, 2, 3 or 4 carbons;

a is an integer from 1-3;

b is an integer from 1-3;

c is an integer from 0-2; and

d is an integer from 0-2, such that (c + d) equals 0-2;

and the pharmaceutically acceptable non-toxic salts thereof which process comprises:

a) treating a compound of the formula

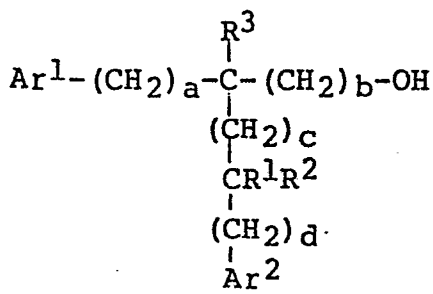

II

wherein:

$Ar^1$, $Ar^2$, $R^1$, $R^2$, $R^3$, a, b, c, d, are as defined herein, with a halogenating agent or a sulfonyl halide, followed by

b) treating the resultant with imidazole;

optionally followed by

c) converting the resulting free base to a pharmaceutically acceptable non-toxic salt.

d) converting a salt of the compound of Formula I to a free base; or

e) converting a salt of the compound of Formula I to another salt.

14. A process for preparing a compound of the formula

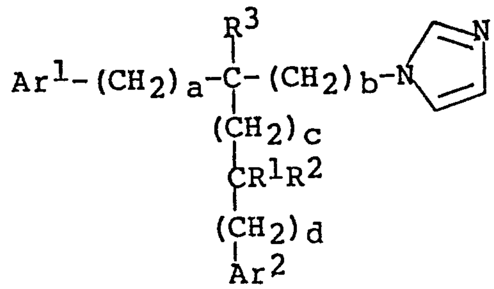

(I)

and the pharmaceutically acceptable non-toxic salts thereof; wherein:

$Ar^1$ and $Ar^2$ are each independently optionally substituted phenyl where substituted means that 1, 2, 3, 4 and/or 5 hydrogens on the phenyl ring are replaced by halo, alkyl, alkoxy, or trifluoromethyl;

$R^1$, $R^2$ and $R^3$ are each independently hydrogen or alkyl where alkyl is a branched or unbranched hydrocarbon residue containing 1, 2, 3 or 4 carbons;

a is an integer from 1-3;

b is an integer from 1-3;

c is an integer from 0-2; and

d is an integer from 0-2, such that (c + d) equals 0-2;

and the pharmaceutically acceptable non-toxic salts thereof which process comprises:

a) reducing one or more compounds selected from the group consisting of:

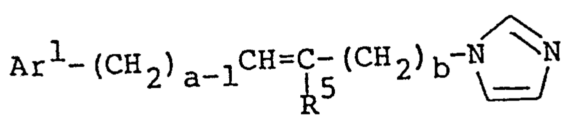

(A)

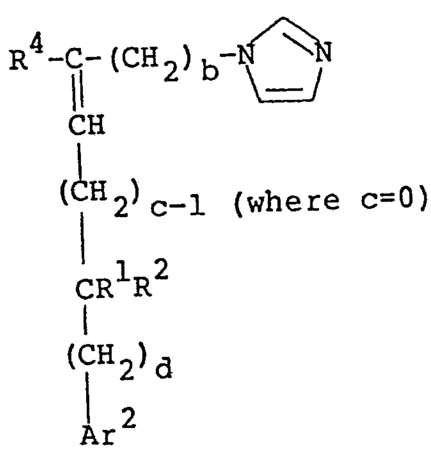

(B)

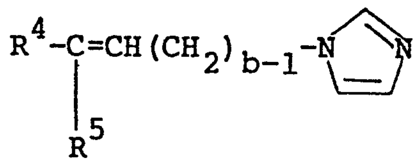

(C)

wherein $Ar^1$, $Ar^2$, $R^1$, $R^2$, a, b, c, and d are as herein defined except that in the compound of formula B, $c \neq 0$, $R^4$ is $Ar^1(CH_2)_a-$, and $R^5$ is $Ar^2(CH_2)_dCR^1R^2(CH_2)_c-$

b) optionally followed by converting the free base of the compound of Formula I to a pharmaceutically acceptable acid addition salt;

c) converting a salt of the compound of Formula I to a free base; or

d) converting a salt of the compound of Formula I to another salt.

0064244

European Patent Office

# EUROPEAN SEARCH REPORT

Application number

EP 82 10 3468

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 530 172 (ROHM & HAAS)<br>----- | | C 07 D 233/58<br>A 61 K 31/415 |
| | | | TECHNICAL FIELDS SEARCHED (Int. Cl. 3) |
| | | | C 07 D 233/00<br>A 61 K 31/00 |

The present search report has been drawn up for all claims

| Place of search | Date of completion of the search | Examiner |
|---|---|---|
| THE HAGUE | 05-08-1982 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82